Europäisches Patentamt

European Patent Office

Office européen des brevets

(19)

(11) Publication number: **0 077 868**

**A1**

## (12) EUROPEAN PATENT APPLICATION

(21) Application number: **81830206.9**

(51) Int. Cl.³: **A 61 F 1/03**

(22) Date of filing: **26.10.81**

(43) Date of publication of application:
04.05.83 Bulletin 83/18

(84) Designated Contracting States:
AT BE CH DE FR GB IT LI LU NL SE

(71) Applicant: Godoli, Nunzio
Via Boccaccini
I-48100 Ravenna(IT)

(71) Applicant: Paribelli, Gianezio
Via Branzanti, 2
I-48100 Ravenna(IT)

(72) Inventor: Godoli, Nunzio
Via Boccaccini
I-48100 Ravenna(IT)

(72) Inventor: Paribelli, Gianezio
Via Branzanti, 2
I-48100 Ravenna(IT)

(74) Representative: Sassatelli, Franco
c/o INIP Ufficio Internazionale Brevetti per Deposito di
Brevetti e Marchi via Mazzini, 170
I-40139 Bologna(IT)

(54) Hip prosthesis as expanding femoral component.

(57) A hip prothesis (3) is provided to fix in the femural articulation (2), with gradual expansion on the wall of excavation by means of screw control.

It shows a top emerging part with articulation conformation with seat for nut settling of screw control which inside in the lower cylindrical part (13), has three longitudinal slots (14). Within the lower cylindrical part (13) a conical, truncated part (4) is axially movable.

When brought upwards, this part (4) causes the progressive diverging of the three relevant expansion sectors (of 13) which block the prothesis in the thigh-bone canal. The integral condition is strengthened by circular retaining reliefs (15), which fit against the cylindrical thigh-bone canal.

./...

FIG.1

- 1 -

"Hip prothesis as expanding femoral component".

The invention refers to a hip prothesis to be fixed in the femoral articulation,with uniformly shared expansion and to be adjusted on the cavity inner wall by means of screw control to act on from the projecting part.

Following arthrosis,ischemical factors of the head,neck fracture,articulation tumours or other,the necessity occurs to replace the coxofemural articulation either partially or totally with a prothesis.For fixing it,several systems are used at present.One system employs acrilic quick settling cement with intense esothermic reaction.The system may turn out inefficient due to the tissue necrosis and give rise to inconveniences of different kind on account to reaction of the organism to cement,such as pressure lowering,psychical turbs and collapsial syndrome.

Another system foresees a boring for adjusting the femural duct and fixing the prothesis in it through a screw device; this system leads the prothesis to stir with the time due to unscrewing.

The invented system permits the solution of the problem by excluding the fixing interposition product and by fitting the prothesis in the femoral canal by incorporating an expanding device which operates with a graduable pressure and uniformly shared effect.This enables fitting integral ly operating of the prothesis to the skeleton avoiding pa rts differently stressed and rejection chances.

In particular,the exclusion of the fixing product helps to avoid complication if the prothesis must replaced,through the difficulty of having to remove the cement from the ar ticulation,or the weakening of the femural cortical,or on account of tissues degenerated by the cement.

The system substantially foresees an envelope with longit udinal development and inside cavity,the top part of which is projecting with an articulation shape,whereas the bott om one is cylindrical with longitudinal fents for giving rise to expansion sectors.A hollow positioning seat is fo reseen above for the control means through a screw axial ly fitted in the cavity binding a truncated cone for long itudinal gliding only which determines the progressive di verging of the expanding sectors by shape contact when li fting,so that these ones block,in an integrally operating way,the prothesis in the femural duct.

The binding condition is strengthened by a system of circ ular horizontal retaining reliefs,externally fitted without interruption on the expansion part;these reliefs are blocking the prothesis in the femural cavity according to a system of circular contact lines with solution of conti nuity,increasing in this way the integrally operating eff ect of composition.

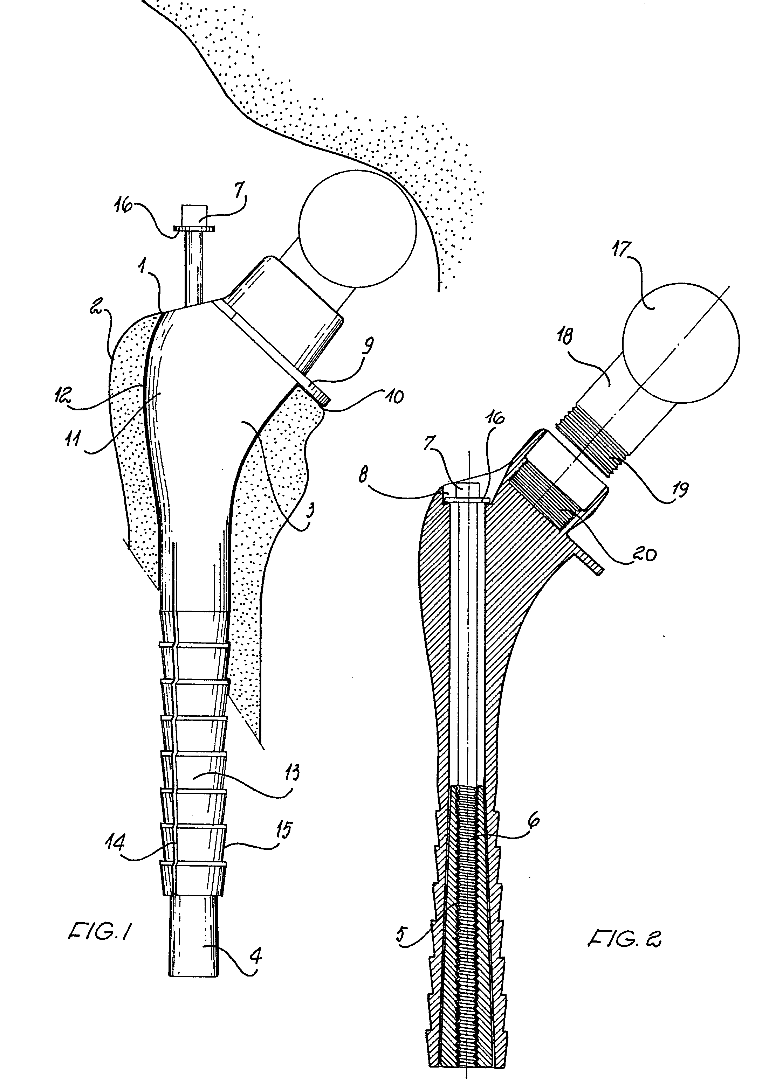

A not limiting version of execution is illustrated by the drawings of Table 1,where Fig.1 is the view of the prothe sis fitted into the femural duct with not activated expan ding device.The settling collar can be noted on the edge of the thigh bone,the control screw nut and the lower cylindrical part with the retention reliefs and from the bottom of which the conical diverging part is projecting.Fig. 2 is the longitudinal section of the prothesis.The version foresees the introduction of the metal part 3 into cavity 1 of the femural articulation 2;the said metal part foresees below the truncated cone shaped part 4 with inside threaded conduit 5 for binding,with axial slide,screw 6 controlled by nut 7 fitted in seat 8 with a view to lifting part 4.Part 3 can be stabilized on the articulation by means slanting collar 9 which is settled on the middle counterfort of the thigh bone 10,while part 11 is set with contact of the inside surface 12 of the femural large trocanther.The cylindrical bottom part 13 shows three longitudinal spaced fents 14 which in opening determine three expansion sectors following the rise of 4.On the outer wall, part 13 foresees a system of retention reliefs 15 without interruption to increase the effect of solidarily operating binding.For settling,the prothesis is at first fitted in the femural conduct,and the expanding effect is activated by acting on the control nut 7 bound in its seat to the rotation of its circular base 16 only,adjusted to cavity 8.The prothesis can be completed with part 17 screwed on the lower cylindrical one 18 threaded on end 19 in coaxial seat 20.

The execution particulars,the control device of the screw, the expanding sectors and everything else in this connection may be executed in a different way,similarly to the materials of possible employ which may be replaced by others suited for the purpose.In particular the spherical head can be otherwise fitted through a kerf system.

CLAIMS.

1)Hip prothesis as expanding femoral component,characteri zed by the fact that foresees an envelope with longitudin al development and inside cavity,the top part of which is projecting with an articulation shape,whereas the bottom one is cylindrical with longitudinal fents for giving rise to expansion sectors.A hollow positioning seat is fore seen above for the control means through a screw axially fitted in the cavity binding a truncated cone for longitu dinal gliding only which determines the progressive diver ging of the expanding sectors by shape contact when lifting,so that these ones block,in an integrally operating way,the prothesis in the femural duct.

2)Hip prothesis as expanding femoral component,according to the previous claim,characterized by the fact that the binding condition is strengthened by a system of circular horizontal retaining reliefs,externally fitted without in terruption on the expansion part;these reliefs are blocking the prothesis in the femural cavity according to a sy stem of circular contact lines with solution of continuity increasing in this way the integrally operating effect of composition.

3)Hip prothesis as expanding femoral component,according to the previous claims,characterized by the fact that the version foresees the introduction of the metal part 3 in- to cavity 1 of the femural articulation 2;the said metal part foresees below the truncated cone shaped part 4 with inside threaded conduit 5 for binding,with axial slide,sc rew 6 controlled by nut 7 fitted in seat 8 with a view to lifting part 4.

0077868

4) Hip prothesis as expanding femoral component, according to the previous claims, characterized by the fact that, part 3 can be stabilized on the articulation by means slanting collar 9 which is settled on the middle counterfort of the thigh bone 10, while part 11 is set with contact of the in side surface 12 of the femural large trocanther.

5) Hip prothesis as expanding femoral component, according to the previous claims, characterized by the fact that the cylindrical bottom part 13 shows three longitudinal spaced fents 14 which in opening determine three expansion secto rs following the rise of 4.

6) Hip prothesis as expanding femoral component, according to the previous claims, characterized by the fact that on the outer wall, part 13 foresees a system of retention rel iefs 15 without interruption to increase the effect of so lidarily operating binding.

7) Hip prothesis as expanding femoral component, according to the previous claims, characterized by the fact that for settling, the prothesis is at first fitted in the femural conduct, and the expanding effect is activated by acting on the control nut bound in its seat to the rotation of its circular base 16 only, adjusted to cavity 8.

8) Hip prothesis as expanding femoral component, according to the previous claims, characterized by the fact that the prothesis can be completed with part 17 screwed on the lo wer cylindrical one 18 threaded on end 19 in coaxial seat 20.

FIG.1    FIG.2

European Patent
Office

**EUROPEAN SEARCH REPORT**

**0077868**

Application number

EP 81 83 0206.9

## DOCUMENTS CONSIDERED TO BE RELEVANT

| Category | Citation of document with indication, where appropriate, of relevant passages | Relevant to claim |
|---|---|---|
| Y | DE - B - 2 247 560 (FISCHER)<br>* claim 1; fig. 1 *<br>& US - A - 3 846 846<br>-- | 1,2 |
| Y | US - A - 3 805 302 (MATHYS)<br>* claim 4; fig. 1 *<br>-- | 1,2 |
| Y | US - A - 3 708 883 (FLANDER)<br>* claim 1; fig. 1 to 3 *<br>-- | 1,2 |
| A | US - A - 4 011 602 (RYBICKI et al. )<br>* claim 1 *<br>-- | 1 |
| A | DE - B1 - 2 338 136 (GEBR. SULZER AG)<br>* claim 1 *<br>-- | 1 |
| A | FR - A2 - 2 349 319 (RAMBERT et al.)<br>* fig. 1, 2 *<br>& US - A - 4 115 875<br>-- | 8 |
| A | US - A - 2 381 050 (HARDINGE)<br>-- | |
| A | DE - B - 2 109 162 (FISCHER)<br>& US - A - 3 760 802<br>---- | |

**CLASSIFICATION OF THE APPLICATION (Int. Cl. ³)**

A 61 F    1/03

**TECHNICAL FIELDS SEARCHED (Int.Cl. ³)**

A 61 B    17/18

A 61 F    1/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| Berlin | 19-05-1982 | KANAL |

EPO Form 1503.1   06.78